# EUROPEAN PATENT APPLICATION

(11) **EP 1 884 883 A2**
(43) Date of publication of application: **06.02.2008**
(21) Application number: 07014718.6
(22) Date of filing: 26.07.2007
(51) Int. Cl.: G06K 9/00

(54) **Three-dimensional circuit board and fingerprint sensor device**

(30) Priority: 31.07.2006 JP 2006208838
(71) Applicant: MATSUSHITA ELECTRIC WORKS, LTD., Kadoma-shi, Osaka 571-8686 (JP)
(72) Inventor: Kobayashi, Mitsuru, Osaka 571-8686 (JP)
(74) Representative: Appelt, Christian W.

(57) **Abstract**

A fingerprint sensor device (A1) is mounted on a printed wiring board (120) by bonding a conductive pattern (2a) formed on a leg portion (2) of a three-dimensional circuit board (B1) and a conductive pattern on the printed wiring board (120) accommodated in a housing (200) of an electronic device with a solder (5). The detecting surface (104) of a fingerprint sensor element (101) is exposed on the surface of the housing (200) through a window hole (201) provided at the housing (200). As compared to the conventional art that utilizes connectors (121) and (122), the detecting surface of the fingerprint sensor element (101) is accurately positioned relative to the window hole (201) provided at the housing (200) of the electronic device.

## Description

### CROSS REFERENCE TO RELATED APPLICATION

This application is based upon and claims the benefit of priority from prior Japanese Patent Application P 2006-208838, filed on July 31, 2006; the entire contents of which are incorporated by reference herein.

### BACKGROUND OF THE INVENTION

### 1. Field of the Invention

The present invention relates to a three-dimensional circuit board for mounting a fingerprint sensor element or a fingerprint sensor that obtains a fingerprint image for fingerprint authentication on the printed wiring board of an electronic device, and a fingerprint sensor device that includes the fingerprint sensor element or the fingerprint sensor and the three-dimensional circuit board.

### 2. Description of the Related Art

Recently, technologies called biometric authentication for identifying individuals with the physical feature amount have been spread instead of password authentication. Examples of the physical feature amount utilized for the biometric authentication include blood vessel (vein), voiceprint, iris, and fingerprint. Fingerprint authentication is widely utilized in cell phones, PDAs, and laptop personal computers because devices for collecting feature amounts are made to be compact and the accuracy of authentication based on such feature amounts is relatively high.

In fingerprints, a large number of linearly raised portions of skin, so-called ridges are gathered to form patterns. While the entire fingerprint is formed concentrically, the respective ridges diverge or terminate in some parts so that complicated patterns are formed. The diverging and terminating portions of the ridges are called fingerprint feature points. According to the basic principal of fingerprint authentication, comparison of consistency of the fingerprint feature points is performed in terms of position, type, and direction.

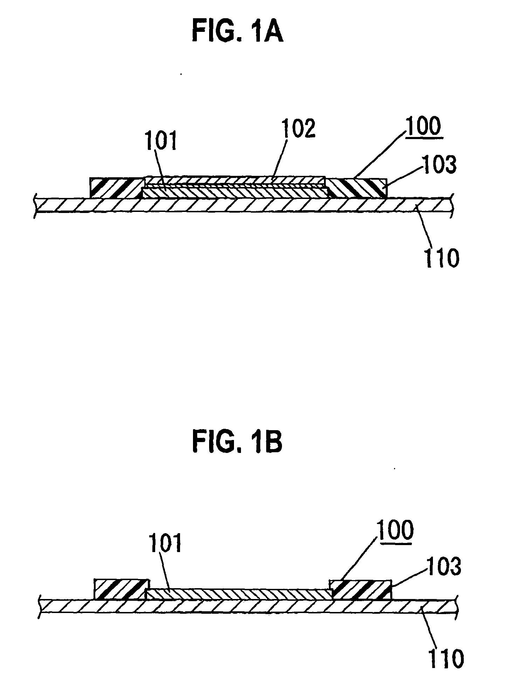

To obtain fingerprint images so as to extract fingerprint feature points, optical and capacitance methods are utilized. Capacitive semiconductor fingerprint sensors are usually utilized in terms of size and cost of devices for obtaining fingerprint images (fingerprint sensors) (see for example, Japanese Patent Application Laid-open Nos. 2005-276217 and 2003-308516). The capacitive semiconductor fingerprint sensor (hereinafter, fingerprint sensor) 100 includes, as shown in Fig. 1A, a fingerprint sensor element 101 on which tens of thousands of electrodes are arranged by processing a semiconductor substrate, a hard protective film 102 which coats the surface of the fingerprint sensor element 101, a signal processing circuit (not shown) which detects the electric charge or electric field amount accumulated in the electrodes depending on the distance to the surface of a finger contacting the detecting surface (surface of the protective film 102) so as to perform signal processing such as amplification and A/D conversion, and a synthetic resin package 103 for shielding the signal processing circuit while the surface of the fingerprint sensor element 101 is exposed through the protective film 102. The fingerprint sensor 100 outputs fingerprint images subjected to signal processing in the signal processing circuit from an output terminal (not shown) provided at the package 103. Fingerprint authentication software obtains the fingerprint images outputted from the fingerprint sensor 100, then extracts, verifies, and authenticates the fingerprint feature points. As shown in Fig. 1B, the protective film 102 may not be provided.

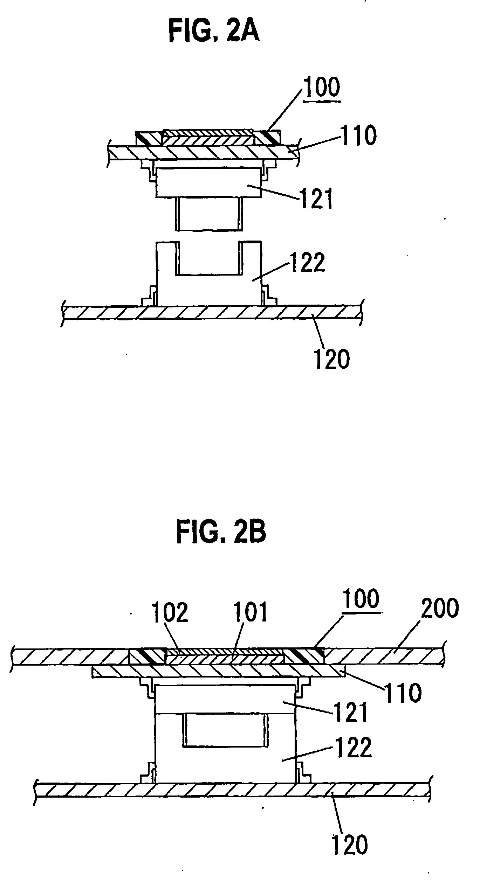

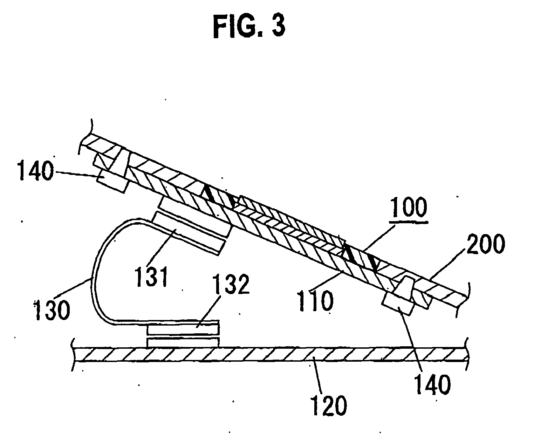

Japanese Patent Application Laid-Open Nos. 2005-276217 and 2003-308516 describe cell phones as an electronic device with the fingerprint sensor. The detecting surface of the fingerprint sensor is exposed on the surface of a housing for the cell phone through a window hole provided at the housing. A conductive pattern formed on a printed wiring board accommodated in the housing must be electrically connected to the fingerprint sensor in order to supply power and obtain fingerprint images. To accomplish such electrical connection, generally, as shown in Figs. 2A and 2B, a printed wiring board 110 on which the fingerprint sensor 100 is mounted is connected directly to the printed wiring board 120 of the electronic device (cell phone) by connectors 121 and 122. Alternatively, connectors 131 and 132 provided at the ends of a flexible substrate 130 are utilized as shown in Fig. 3.

If the connectors 121 and 122 for connecting substrates are utilized as in the conventional case of Figs. 2A and 2B, the fitting height of the connectors 121 and 122 must be 1 mm or higher in order to ensure connection reliability. An error of ±0. 1 to 0. 3 mm should be considered. As shown in Figs. 2A and 2B, the distance between the mounting surface of the printed wiring board 120 and the detecting surface of the fingerprint sensor 100 hardly matches the distance between the mounting surface of the printed wiring board 120 and the surface of the housing 200 for the electronic device (i.e., positioning is difficult). In particular, positioning is more difficult in thin electronic devices with a shorter distance between the mounting surface of the printed wiring board 120 and the surface of the housing 200.

If the mounting surface of the printed wiring board 120 accommodated in the housing 200 is not parallel to the surface of the housing 200 with the exposed detecting surface of the fingerprint sensor 100, the connectors 131 and 132 at the ends of the flexible substrate 130 are utilized as in the conventional case of Fig. 3. The number of connectors 131 and 132 is twice the conventional case of Figs. 2A and 2B. An additional screw 140 for fixing the printed wiring board 110 on which the fingerprint sensor 100 is mounted to the housing 200 is required. The number of components is thus increased, and effort and cost for assembling are also increased.

### SUMMARY OF THE INVENTION

The present invention has been achieved in view of the above circumstances, and an object of the invention is to provide a three-dimensional circuit board and a fingerprint sensor device that position the detecting surface of a fingerprint sensor element or a fingerprint sensor accurately relative to a window hole provided at a housing for an electronic device.

A first aspect of the present invention provides a three-dimensional circuit board on which a fingerprint sensor element that converts a fingerprint image obtained from its flat detecting surface into an electric signal for outputting is mounted, the detecting surface being exposed to the outside of a housing of an electronic device through a window hole provided at the housing, and which is mounted on a printed wiring board accommodated in the housing, the three-dimensional circuit board comprising: a synthetic resin molded component that includes a mounting portion in which a concave portion for accommodating the fingerprint sensor element, and at least one leg portion whose end portion is connected to the mounting portion and whose other end portion is mounted on the printed wiring board, the mounting portion and the leg portion being formed integrally; and a conductive body that is provided on the synthetic resin molded component, and electrically connects an output terminal of the fingerprint sensor element mounted on the mounting portion and a conductive pattern formed on the printed wiring board.

A second aspect of the present inventionprovides a fingerprint sensor device comprising: a fingerprint sensor element that converts a fingerprint image obtained from its flat detecting surface into an electric signal for outputting; and a three-dimensional circuit board on which the fingerprint sensor element is mounted, the detecting surface being exposed to the outside of a housing of an electronic device through a window hole provided at the housing, and which is mounted on a printed wiring board accommodated in the housing, the three-dimensional circuit board comprising: a synthetic resin molded component that includes a mounting portion at which a concave portion for accommodating the fingerprint sensor element, and at least one leg portion whose end portion is connected to the mounting portion and whose other end portion is mounted on the printed wiring board, the mounting portion and the leg portion being formed integrally; and a conductive body that is provided on the synthetic resin molded component and electrically connects an output terminal of the fingerprint sensor element mounted on the mounting portion and a conductive pattern formed on the printed wiring board.

In the first and second aspects, the three-dimensional circuit board can include a metal film for electromagnetic shield that is extended from a peripheral portion of the concave portion to a outer surface of the leg portion.

In the first and second aspects, a circuit component for performing signal processing to an output from the fingerprint sensor element is mounted on a region surrounded by the metal film within the leg portion.

In the first and second aspects, a surface of the mounting portion at which the concave portion is provided is formed so as to conform to a surface of the housing on the periphery of the window hole.

In the first and second aspects, the three-dimensional circuit board can include a pair of guide portions that oppose with each other on the surface of the mounting portion with the detecting surface interposed therebetween, protrude further than the detecting surface through the window hole, and position a finger relative to the detecting surface.

A third aspect of the present invention provides a three-dimensional circuit board that supports a fingerprint sensor which accommodates a fingerprint sensor element that converts a fingerprint image obtained from its flat detecting surface into an electric signal for outputting in a package, the detecting surface being exposed to the outside of a housing of an electronic device through a window hole provided at the housing, and that is mounted on a printed wiring board accommodated in the housing, the three-dimensional circuit board comprising: at least one support which supports the fingerprint sensor at its end portion and whose other end portion is mounted on the printed wiring board; and a conductive body that is provided on the support and electrically connects an output terminal of the fingerprint sensor and a conductive pattern formed on the printed wiring board.

A fourth aspect of the present invention provides a fingerprint sensor device comprising: a fingerprint sensor that accommodates a fingerprint sensor element which converts a fingerprint image obtained from its flat detecting surface into an electric signal for outputting in a package; and a three-dimensional circuit board that supports the fingerprint sensor, the detecting surface being exposed to the outside of a housing of an electronic device through a window hole provided at the housing, and that is mounted on a printed wiring board accommodated in the housing, the three-dimensional circuit board comprising: at least one support which supports the fingerprint sensor at its end portion and whose other end portion is mounted on the printed wiring board; and a conductive body that is provided on the support and electrically connects an output terminal of the fingerprint sensor and a conductive pattern formed on the printed wiring board.

### BRIEF DESCRIPTION OF THE DRAWINGS

Figs. 1A and 1B are cross-sectional views of a fingerprint sensor;
Figs. 2A and 2B are cross-sectional views of a conventional art;
Fig. 3 is a cross-sectional view of another conventional art;
Fig. 4A is a perspective view of a three-dimensional circuit board according to a first embodiment of the present invention, seen from the side of a mounting portion;
Fig. 4B is a perspective view of the three-dimensional circuit board according to the first embodiment, seen from the side of a leg portion;
Fig. 4C is a cross-sectional view of a fingerprint sensor device according to the first embodiment;
Fig. 5A is a cross-sectional view of a fingerprint sensor device that includes a three-dimensional circuit board according to a modification of the first embodiment;
Fig. 5B is a cross-sectional view of a fingerprint sensor device that includes a three-dimensional circuit board according to a modification of the first embodiment;
Fig. 6 is a cross-sectional view of a fingerprint sensor device according to a second embodiment of the present invention;
Fig. 7A is a plan view of a fingerprint sensor device that includes a three-dimensional circuit board according to a modification of the second embodiment;
Fig. 7B is a side view of a fingerprint sensor device that includes a three-dimensional circuit board according to a modification of the second embodiment;
Fig. 7C is a side view illustrating a state that a finger is scanned on the detecting surface of the fingerprint sensor device that includes the three-dimensional circuit board according to the modification of the second embodiment;
Fig. 8A is a perspective view of a three-dimensional circuit board according to a third embodiment of the present invention;
Fig. 8B is a cross-sectional view of a fingerprint sensor device according to the third embodiment; and
Fig. 9 is a cross-sectional view of a fingerprint sensor device according to a fourth embodiment of the present invention.

### DETAILED DESCRIPTION OF THE PREFERRED EMBODIMENTS

Preferred embodiments of the present invention are explained below with reference to the accompanying drawings. Because a fingerprint sensor 100 and a fingerprint sensor element 101 according to the respective embodiments have the same configuration as in the conventional arts, the same reference numerals are used to designate the same components, and their descriptions will be omitted.

### (First Embodiment)

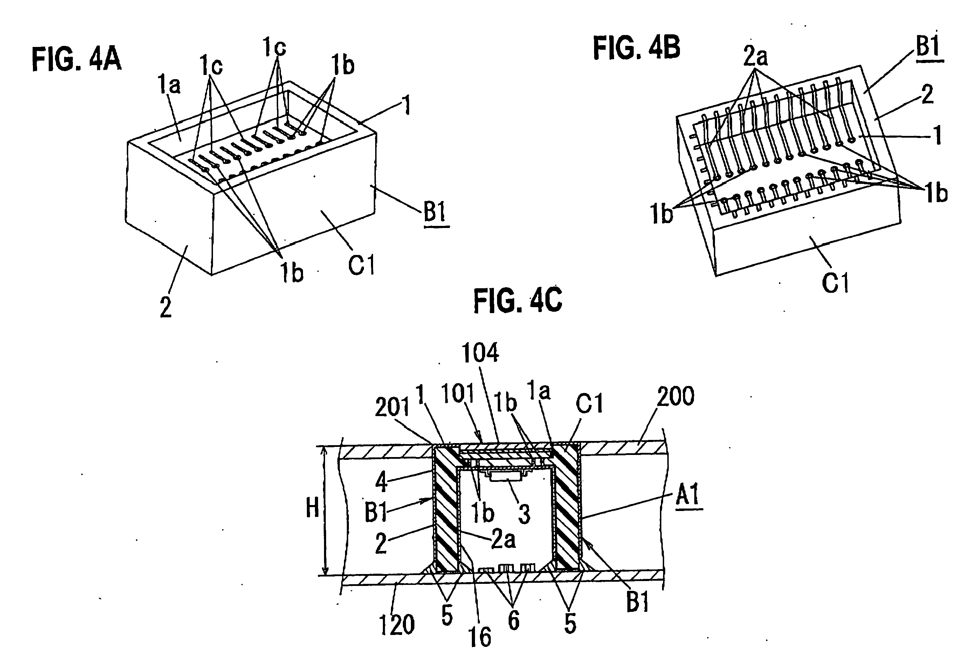

A first embodiment of the present invention will be described first. A fingerprint sensor device A1 of the first embodiment includes, as shown in Figs. 4A, 4B, and 4C, a three-dimensional circuit board B1 and the fingerprint sensor element 101 mounted on the three-dimensional circuit board B1.

The three-dimensional circuit board B1 is of a rectangular parallelepiped box shape and includes a synthetic resin molded component C1 that is obtained by forming a mounting portion 1 and a rectangular cylindrical leg portion 2 connected to the bottom surface of the mounting portion 1 integrally.

A concave portion 1a for accommodating the fingerprint sensor element 101 is provided on the top surface of the mounting portion 1. A number of through-holes 1b are penetrated in two rows through the bottom surface of the concave portion 1a along the longitudinal direction of the mounting portion 1. A conductive body (a conductive pattern) 1c made of metal film extends from each of the through-holes 1b (see Fig. 4A).

The fingerprint sensor element 101 is accommodated in the concave portion 1a and an output terminal of the fingerprint sensor element 101 is electrically connected to each conductive pattern 1c. The fingerprint sensor element 101 is thus mounted on the mounting portion 1.

As shown in Fig. 4B, a plurality of conductive patterns 2a extend from the through-holes 1b on the bottom surface of the mounting portion 1 (the surface opposing the bottom surface of the concave portion 1a).The conductive pattern 2a, the through-hole 1b, and the conductive body 1c constitute a conductive body 16. The conductive body 16 electrically connects the output terminal of the fingerprint sensor element 101 and the conductive pattern formed on the printed wiring board 120. The conductive pattern 2a extends from the through-hole 1b toward the bottom surface of the leg portion 2 (the surface to be mounted on a printed wiring board 120). An electronic component (a circuit component) 3 that configures a signal processing circuit for detecting the electric charge or electric field amount accumulated in the electrodes for the fingerprint sensor element 101 to perform signal processing such as amplification and A/D conversion is mounted on the bottom surface of the mounting portion 1 (see Fig. 4C). A metal film 4 for electromagnetic shield is formed all over from the peripheral portion of the concave portion 1a on the mounting portion 1 to the outer surface of the leg portion 2.

A method for forming the conductive patterns 1c and 2a on the synthetic resin molded component C1 that is obtained by forming the mounting portion 1 and the leg portion 2 integrally so as to make the three-dimensional circuit board B1 will be described in brief. A plated base layer made of a thin film of conductive material (e. g. , copper) is formed on the surface of the synthetic resin molded component C1. A laser beam is applied to the plated base layer formed to remove the plated base layer at the outline of the required conductive pattern. A required part of the remaining plated base layer laser not subjected to irradiation by the laser beam is then electroplated, so that the conductive patterns 1c and 2a are formed.

As shown in Fig. 4C, the fingerprint sensor device A1 with the above configuration is mounted on the printed wiring board 120 by bonding the conductive pattern 2a formed on the leg portion 2 and the conductive pattern of the printed wiring board 120 accommodated in the housing 200 of the electronic device with a solder 5. The detecting surface 104 of the fingerprint sensor element 101 is exposed on the surface of the housing 200 through the window hole 201 provided at the housing 200. The dimensional accuracy of the synthetic resin molded component C1 that configures the three-dimensional circuit board B1 is higher than that of the fitting height of the connectors 121 and 122 (e.g., ±0.02 to 0.1 mm). Compared to the conventional art utilizing the connectors 121 and 122, the detecting surface 104 of the fingerprint sensor element 101 is positioned accurately relative to the window hole 201 provided at the housing 200 of the electronic device. To electrically connect the fingerprint sensor element 101 to the conductive pattern of the printed wiring board 120, connecting components including such connectors are not used. Cost reduction and miniaturization due to a reduction in the number of components are accomplished. The fingerprint sensor element 101 mounted in the concave portion 1a of the mounting portion 1, the electronic component 3 mounted on the bottom surface of the mounting portion 1, and electronic components 6 mounted on the mounting surface of the printed wiring board 120 within the leg portion 2 are electromagnetically shielded with the metal film 4 extended from the peripheral portion of the concave portion 1a of the mounting portion 1 to the outer surface of the leg portion 2. Influences of static noises outputted from fingertips are thus reduced.

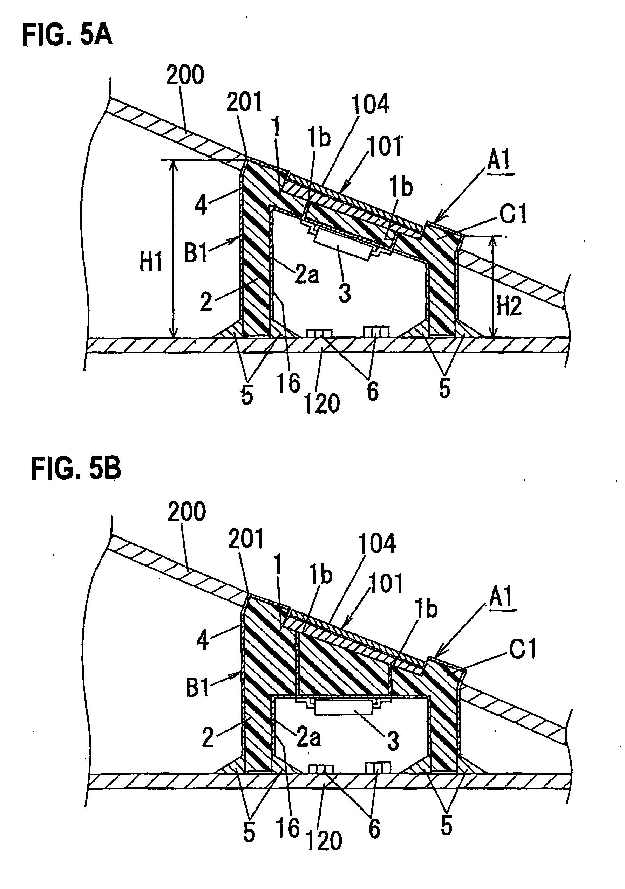

Referring to Figs. 4A, 4B, and 4C, while the height H of the leg portion 2 is uniform because the surface of the housing 200 is substantially parallel to the mounting surface of the printed wiring board 120, the height needs not to be uniform. For example, if the surface of the housing 200 is inclined relative to the mounting surface of the printed wiring board 120, as shown in Figs. 5A and 5B, the height of the leg portion 2 is gradually reduced from the maximum height value H1 to the minimum height value H2 so that the mounting portion 1 fitted in the window hole 201 is inclined relative to the mounting surface of the printed wiring board 120 at the same angle as the surface of the housing 200. Because the synthetic resin molded component C1 formed by the mounting portion 1 and the leg portion 2 is made in a form depending on the relative positional relationship between the housing 200 and the printed wiring board 120, the synthetic resin molded component C1 is utilized for electronic devices with various configurations. As shown in Fig. 5A, when the bottom surface of the mounting portion 1 on which the electronic component 3 is mounted is parallel to the surface of the housing 200, it is difficult to mount the electronic component 3 from the leg portion 2 side because of the inclined mounting surface. As shown in Fig. 5B, when the bottom surface of the mounting portion 1 is made to be parallel to the mounting surface of the leg portion 2 mounted on the printed wiring board 120, the electronic component 3 is mounted easily on the bottom surface of the mounting portion 1 without obstruction of the leg portion 2.

### (Second Embodiment)

A second embodiment of the present invention will be described. The configuration of a fingerprint sensor device A2 of the second embodiment is the same as in the first embodiment except for the configuration of a three-dimensional circuit board B2 including a synthetic resin molded component C2. The same reference numerals are used to designate the same components as in the first embodiment and their descriptions will be omitted.

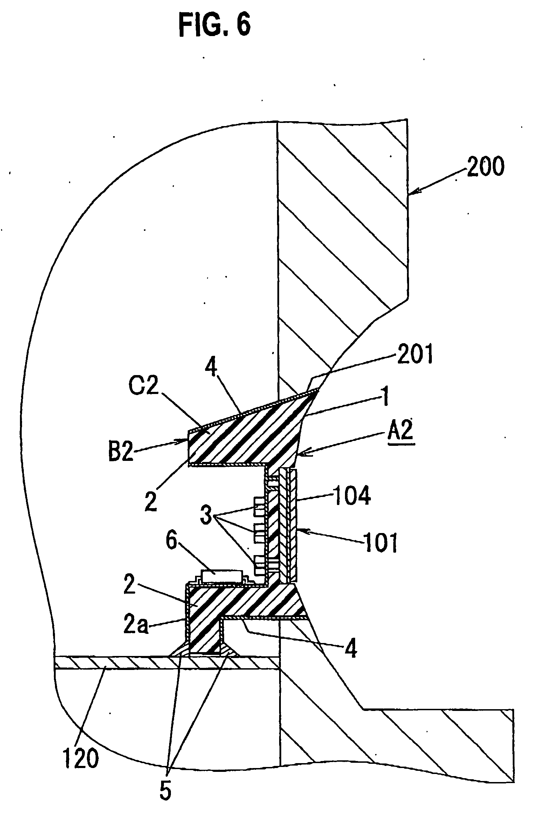

According to the three-dimensional circuit board B2 of the second embodiment, as shown in Fig. 6, the surface of the mounting portion 1 at which the concave portion 1a is provided is formed in a curve so as to conform to the surface (curved surface) on the periphery of the window hole 201 of the housing 200 for the electronic device. The printed wiring board 120 is arranged so as to be perpendicular to the housing 200, and an end of the leg portion 2 is formed in an approximately L-shaped configuration. The electronic component 6 is mounted on the inner side surface of the leg portion 2.

The mounting portion 1 is fitted in the window hole 201 provided at the housing 200, and the conductive pattern 2a on the approximately L-shaped leg portion 2 and the conductive pattern (not shown) of the printed wiring board 120 are bonded with the solder 5. The fingerprint sensor device A2 is thus mounted on the printed wiring board 120. Because the surface of the mounting portion 1 is formed in a curve so as to conform to the surface of the housing 200, the aesthetic appearance of the electronic device is not spoiled. At least the surface of the mounting portion 1 exposed to the outside of the housing 200 through the window hole 201 is preferably colored with the same color as the surface on the periphery of the window hole 201 provided at the housing 200.

A sweep-type fingerprint sensor element that scans fingers on the detecting surface 104 so as to detect fingerprint images is widely utilized in cell phones because of its compact size as compared to a non-sweep type (so-called surface type) sensor. When the fingerprint sensor devices A1, A2 are configured by mounting the sweep fingerprint sensor element 101 on the three-dimensional circuit boards B1, B2 of the first and second embodiments, fingers may be deviated in a direction perpendicular to the scanning direction at the time of scanning the fingers on the detecting surface 104, so that the accuracy of detecting fingerprint images maybe degraded.

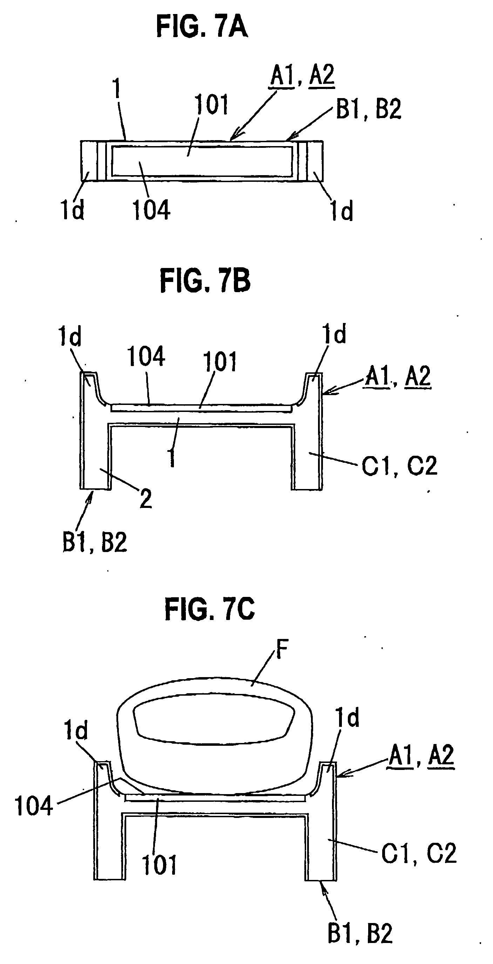

As shown in Figs. 7A, 7B, and 7C, a pair of guide portions 1d is provided. The respective guide portions 1d oppose with each other on the surface of the mounting portion 1 with the detecting surface 104 of the fingerprint sensor element 101 therebetween and protrude further than the detecting surface 104 through the window hole 201. If a finger F to be scanned on the detecting surface 104 is positioned by the pair of guide portions 1d in a direction perpendicular to the scanning direction, the accuracy of detecting the fingerprint image with the sweep type fingerprint sensor element 101 is improved.

### (Third Embodiment)

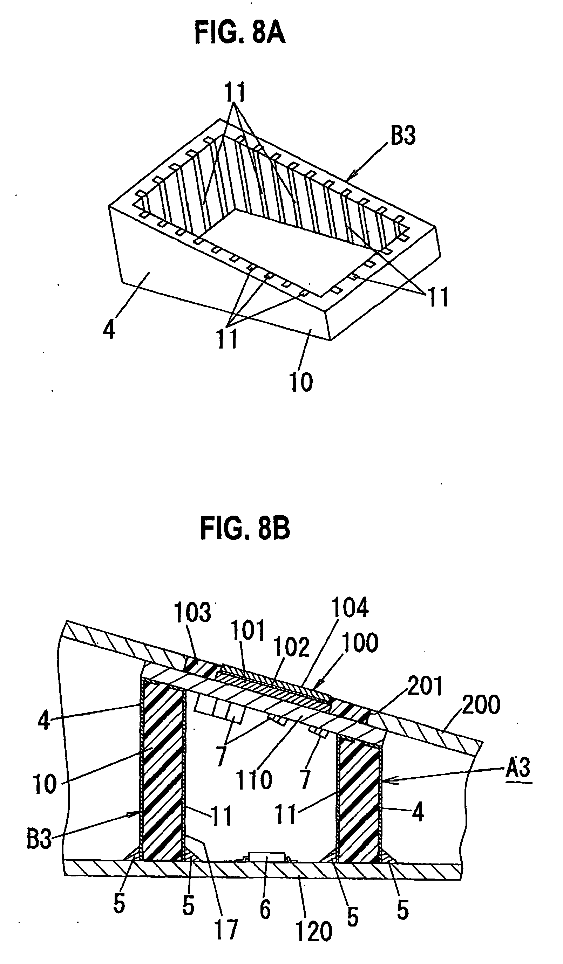

A third embodiment of the present invention will be described. According to the first and second embodiments, the fingerprint sensor devices A1, A2 are configured by mounting the fingerprint sensor element 101 on the mounting portions 1 of the three-dimensional circuit boards B1, B2. According to the third embodiment, as shown in Figs. 8A and 8B, a fingerprint sensor device A3 is configured by the fingerprint sensor 100 mounted on the printed wiring board 110 and a three-dimensional circuit board B3 that supports the fingerprint sensor 100 via the printed wiring board 110 and electrically connects the conductive pattern (not shown) of the printed wiring board 110 and the conductive pattern (not shown) of the printed wiring board 120 accommodated in the housing 200 of the electronic device.

The fingerprint sensor 100 includes, as described in the related arts, the fingerprint sensor element 101, the hard protective film 102 for coating the surface of the fingerprint sensor element 101, the signal processing circuit (not shown) which detects the electric charge or electric field amount accumulated in electrodes depending on the distance to a finger contacting the detecting surface 104 so as to perform signal processing such as amplification and A/D conversion, and the synthetic resin package 103 which shields the signal processing circuit while the surface of the fingerprint sensor element 101 is exposed through the protective film 102. The fingerprint sensor 100 is mounted on one mounting surface of the printed wiring board 110. Electronic components 7 that configure a circuit for verifying and authenticating fingerprint images outputted from the fingerprint sensor 100 are mounted on the other mounting surface of the printed wiring board 110.

The three-dimensional circuit board B3 includes, as shown in Fig. 8A, a support 10 which is a synthetic resin molded component formed in a rectangular cylindrical shape, and a plurality of conductive patterns 11 that are formed at the end surfaces and the inner peripheral surface of the support 10 and electrically connect the conductive pattern (not shown) formed on the printed wiring board 110 and the conductive pattern (not shown) formed on the printed wiring board 120. The conductive pattern 11 and the conductive pattern formed on the printed wiring board 110 constitute a conductive body 17. The conductive body 17 electrically connects the output terminal of the fingerprint sensor 100 and the conductive pattern formed on the printed wiring board 120. The surface of the support 10 that supports the printed wiring board 110 (top surface in Figs. 8A and 8B) is inclined relative to the surface to be mounted on the printed wiring board 120 (bottom surface in Figs. 8A and 8B) . The metal film 4 for electromagnetic shield is formed on the entire peripheral surface of the support 10.

As shown in Fig. 8B, the fingerprint sensor device A3 with the above configuration is mounted on the printed wiring board 120 by bonding the conductive pattern 11 formed on the support 10 and the conductive pattern of the printed wiring board 120 accommodated in the housing 200 of the electronic device with the solder 5. The fingerprint sensor 100 is fitted in the window hole 201 provided at the housing 200 and the detecting surface 104 of the fingerprint sensor element 101 is exposed on the surface of the housing 200 through the window hole 201. As in the first embodiment, the detecting surface 104 of the fingerprint sensor element 101 is accurately positioned relative to the window hole 201 provided at the housing 200 of the electronic device by the three-dimensional circuit board B3. Because connecting components including connectors are not used to electrically connect the fingerprint sensor element 101 to the conductive pattern of the printed wiring board 120, cost reduction and miniaturization due to a reduction in the number of components are realized. The electronic components 7 mounted on the printed wiring board 110 and the electronic component 6 mounted on the mounting surface of the printed wiring board 120 within the three-dimensional circuit board B3 are electromagnetically shielded by the metal film 4 on the outer peripheral surface of the support 10. Influences of static noises outputted from fingertips are thus reduced. Because the existing fingerprint sensor 100 is used as it is, a further cost reduction is realized.

### (Fourth Embodiment)

A fourth embodiment of the present invention will be described. A fingerprint sensor device A4 of the fourth embodiment is the same as in the third embodiment except for the configuration of a three-dimensional circuit board B4. Thus, the same reference numerals are used to designate the same components as in the third embodiment and their descriptions will be omitted.

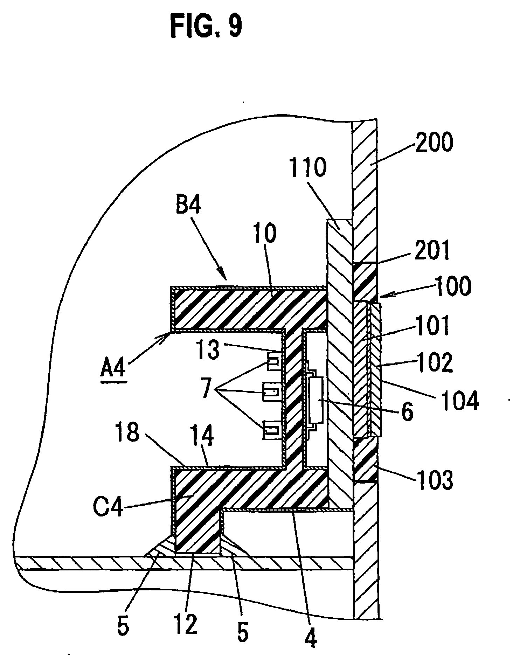

The synthetic resin molded component C4 of the three-dimensional circuit board B4 includes, as shown in Fig. 9, the rectangular cylindrical support 10, a convex portion 12 which protrudes from an end of the support 10 in a direction perpendicular to the axial direction of the support 10, and a planar component mounting portion 13 which blocks the inside of the support 10. A conductive pattern 14 and a metal film 15 for electromagnetic shield are formed on the surfaces of the support 10, the convex portion 12, and the component mounting portion 13. The printed wiring board 110 on which the fingerprint sensor 100 is mounted is mounted on the other end of the support 10. The electronic components 6 and 7 are mounted on the component mounting portion 13.

The fingerprint sensor device A4 with the above configuration is mounted on the printed wiring board 120 by bonding the conductive pattern 14 formed on the convex portion 12 and the conductive pattern of the printed wiring board 120 accommodated in the housing 200 of the electronic device with the solder 5. The fingerprint sensor 100 is fitted in the window hole 201 of the housing 200 which is perpendicular to the printed wiring board 120 and the detecting surface 104 of the fingerprint sensor element 101 is exposed on the surface of the housing 200 through the window hole 201. The conductive pattern 14 and the conductive pattern formed on the printed wiring board 110 constitute a conductive body 18. The conductive body 18 electrically connects the output terminal of the fingerprint sensor 100 and the conductive pattern formed on the printed wiring board 120

According to the fourth embodiment, as in the third embodiment, the detecting surface 104 of the fingerprint sensor element 101 is positioned accurately relative to the window hole 201 provided at the housing 200 of the electronic device by the three-dimensional circuit board B4. Connecting components including connectors are not used to electrically connect the fingerprint sensor element 101 to the conductive pattern of the printed wiring board 120. Cost reduction and miniaturization due to a reduction in the number of components are realized. Because the electronic components 6 and 7 mounted on the component mounting portion 13 of the three-dimensional circuit board B4are electromagnetically shielded by the metal film 15, influences of static noises outputted from fingertips are reduced. The existing fingerprint sensor 100 is used as it is in the fourth embodiment, which leads to a further cost reduction.

Effects of the present invention are as follows.

According to the present invention, a three-dimensional circuit board which includes a mounting portion and a leg portion is formed integrally as a synthetic resin molded component. The detecting surface of a fingerprint sensor element mounted on the mounting portion is exposed to the outside of housing of an electronic device through a window hole provided at the housing. The leg portion is mounted on a printed wiring board accommodated in the housing of the electronic device. As compared to conventional arts utilizing connectors, the detecting surface of the fingerprint sensor element is accurately positioned relative to the window hole provided at the housing of the electronic device. Cost reduction and miniaturization due to a reduction in the number of components are realized.

According to the present invention, the fingerprint sensor element is electromagnetically shielded by a metal film extended from the peripheral portion of a concave portion to the outer surface of the leg portion. Influences of static noises outputted from the human body are thus reduced.

According to the present invention, circuit components for performing signal processing to outputs of the fingerprint sensor element are also electromagnetically shielded. Influences of static noises are reduced.

According to the present invention, the surface of the mounting portion at which the concave portion is provided is formed so as to conform to the surface of housing of the electronic device on the periphery of the window hole. The aesthetic appearance of the electronic device is not spoiled.

According to the present invention, a finger is positioned relative to the detecting surface of the fingerprint sensor element by a pair of guide portions. The accuracy of the fingerprint image is improved.

According to the present invention, a three-dimensional circuit board which has a support for supporting the fingerprint sensor is formed as the synthetic resin molded component. The detecting surface of the fingerprint sensor supported by the support is exposed to the outside of housing of the electronic device through the window hole provided at the housing. The support is mounted on the printed wiring board accommodated in the housing of the electronic device. As compared to conventional arts utilizing connectors, the detecting surface of the fingerprint sensor is accurately positioned relative to the window hole provided at the housing of the electronic device. Cost reduction and miniaturization due to a reduction in the number of components are realized.

While the embodiment of the present invention has been describedabove, the invention is not limited to the above embodiments and changes and modifications can be made within the scope of the gist of the present invention.

## Claims

1. A three-dimensional circuit board on which a fingerprint sensor element (101) that converts a fingerprint image obtained from its flat detecting surface (104) into an electric signal for outputting is mounted, the detecting surface (104) being exposed to the outside of a housing (200) of an electronic device through a window hole (201) provided at the housing (200), and which is mounted on a printed wiring board (120) accommodated in the housing (200),
the three-dimensional circuit board comprising:
a synthetic resin molded component (C1,C2) that includes a mounting portion (1) in which a concave portion (1a) for accommodating the fingerprint sensor element (110), and at least one leg portion (2) whose end portion is connected to the mounting portion (1) and whose other end portion is mounted on the printed wiring board (120), the mounting portion (1) and the leg portion (2) being formed integrally; and
a conductive body (16,17) that is provided on the synthetic resin molded component (C1,C2), and electrically connects an output terminal of the fingerprint sensor element (101) mounted on the mounting portion (1) and a conductive pattern formed on the printed wiring board (120).

2. The three-dimensional circuit board according to claim 1 further comprising a metal film (4) for electromagnetic shield that is extended from a peripheral portion of the concave portion (1a) to a outer surface of the leg portion (2).

3. The three-dimensional circuit board according to claim 2, wherein a circuit component (3) for performing signal processing to an output from the fingerprint sensor element (101) is mounted on a region surrounded by the metal film (4) within the leg portion (2).

4. The three-dimensional circuit board according to claim 1, wherein a surface of the mounting portion (1) at which the concave portion (1a) is provided is formed so as to conform to a surface of the housing (200) on the periphery of the window hole (201).

5. The three-dimensional circuit board according to claim 1 further comprising a pair of guide portions (1d) that oppose with each other on the surface of the mounting portion (1) with the detecting surface (104) interposed therebetween, protrude further than the detecting surface (104) through the window hole (201), and position a finger (F) relative to the detecting surface (104).

6. A fingerprint sensor device comprising:
a fingerprint sensor element (101) that converts a fingerprint image obtained from its flat detecting surface (104) into an electric signal for outputting; and
a three-dimensional circuit board (B1,B2) on which the fingerprint sensor element (101) is mounted, the detecting surface (104) being exposed to the outside of a housing (200) of an electronic device through a window hole (201) provided at the housing (200), and which is mounted on a printed wiring board (120) accommodated in the housing (200),
the three-dimensional circuit board (B1,B2) comprising:
a synthetic resin molded component (C1,C2) that includes a mountingportion (1) at which a concave portion (1a) for accommodating the fingerprint sensor element (101), and at least one leg portion (2) whose end portion is connected to the mounting portion (1) and whose other end portion is mounted on the printed wiring board (120), the mounting portion (1) and the leg portion (2) being formed integrally; and
a conductive body (16, 17) that is provided on the synthetic resin molded component (C1,C2) and electrically connects an output terminal of the fingerprint sensor element (101) mounted on the mounting portion (1) and a conductive pattern formed on the printed wiring board (120).

7. The fingerprint sensor device according to claim 6, wherein the three-dimensional circuit board (B1,B2) further comprises a metal film (4) for electromagnetic shield that is extended from a peripheral portion of the concave portion (1a) to a outer surface of the leg portion (2).

8. The fingerprint sensor device according to claim 7, wherein a circuit component (3) for performing signal processing to an output from the fingerprint sensor element (101) is mounted on a region surrounded by the metal film (4) within the leg portion (2).

9. The fingerprint sensor device according to claim 6, wherein a surface of the mounting portion (1) at which the concave portion (1a) is provided is formed so as to conform to a surface of the housing (200) on the periphery of the window hole (201).

10. The fingerprint sensor device according to claim 6, wherein the three-dimensional circuit board (B2) further comprises a pair of guide portions (1d) that oppose with each other on the surface of the mounting portion (1) with the detecting surface (104) interposed therebetween, protrude further than the detecting surface (104) through the window hole (201), and position a finger (F) relative to the detecting surface (104).

11. Athree-dimensional circuit board that supports a fingerprint sensor (100) which accommodates a fingerprint sensor element (101) that converts a fingerprint image obtained from its flat detecting surface (104) into an electric signal for outputting in a package (103), the detecting surface (104) being exposed to the outside of a housing (200) of an electronic device through a window hole (201) provided at the housing (200), and that is mounted on a printed wiring board (120) accommodated in the housing (200),
the three-dimensional circuit board comprising:
at least one support (10) which supports the fingerprint sensor (100) at its end portion and whose other end portion is mounted on the printed wiring board (120); and
a conductive body (17,18) that is provided on the support (10) and electrically connects an output terminal of the fingerprint sensor (100) and a conductive pattern formed on the printed wiring board (120).

12. A fingerprint sensor device comprising:
a fingerprint sensor (100) that accommodates a fingerprint sensor element (101) which converts a fingerprint image obtained from its flat detecting surface (104) into an electric signal for outputting in a package (103); and
a three-dimensional circuit board (B3, B4) that supports the fingerprint sensor (100), the detecting surface (104) being exposed to the outside of a housing (200) of an electronic device through a window hole (201) provided at the housing (200), and that is mounted on a printed wiring board (120) accommodated in the housing (200), the three-dimensional circuit board (B3,B4) comprising:
at least one support (10) which supports the fingerprint sensor (100) at its end portion and whose other end portion is mounted on the printed wiring board (120); and
a conductive body (17,18) that is provided on the support (10) and electrically connects an output terminal of the fingerprint sensor (100) and a conductive pattern formed on the printed wiring board (120).
